# EUROPÄISCHE PATENTSCHRIFT

(11) **EP 2 707 352 B1**
(45) Veröffentlichungstag und Bekanntmachung des Hinweises auf die Patenterteilung: **14.10.2015**
(21) Anmeldenummer: 12718179.0
(22) Anmeldetag: 25.04.2012
(51) Int. Cl.: C07C 45/74, C07C 49/603, C07C 45/82, C07C 45/85

(54) **VERFAHREN ZUR HERSTELLUNG VON ISOPHORON IN GEGENWART MINDESTENS EINES ENTSCHÄUMERS IN DER ABWASSERKOLONNE IM AUFARBEITUNGSTEIL**
PROCESS FOR PREPARING ISOPHORONE IN THE PRESENCE OF AT LEAST ONE DEFOAMER IN THE WASTEWATER COLUMN IN THE WORKUP SECTION
PROCÉDÉ POUR LA PRÉPARATION D'ISOPHORONE EN PRÉSENCE D'AU MOINS UN AGENT ANTIMOUSSE DANS LA COLONNE DES EAUX USÉES DANS LA PARTIE TRAITEMENT

(30) Priorität: 13.05.2011 DE 102011075777
(43) Veröffentlichungstag der Anmeldung: 19.03.2014
(73) Patentinhaber: Evonik Degussa GmbH, 45128 Essen (DE)
(72) Erfinder: ORSCHEL, Matthias, 48149 Münster (DE); JANSEN, Robert, 46244 Bottrop (DE); MAIER, Martin, 44625 Herne (DE); NITZ, Jörg-Joachim, 45257 Essen (DE); SCHWARZ, Markus, 45721 Haltern am See (DE)
(86) Internationale Anmeldenummer: PCT/EP2012/057562
(87) Internationale Veröffentlichungsnummer: WO 2012/156187

(56) Entgegenhaltungen:
- US-A- 5 352 839

## Beschreibung

Die Erfindung betrifft ein Verfahren zur Herstellung von Isophoron (3,5,5-Trimethyl-2-cyclohexen-1-on) in Gegenwart mindestens eines Entschäumers in der Abwasserkolonne im Aufarbeitungsteil.

Isophoron wird unter anderem als hochsiedendes Lösemittel in der Lack-, Druckfarben-, Klebstoff- und der Pflanzenschutzmittel-Industrie verwendet. Nach dem bekannten Stand der Technik kann das Isophoron weiter verarbeitet werden bspw. zu Isophoronnitril, Isophorondiamin, Isophorondiisocyanat oder Keto-Isophoron.

Bei Isophoron handelt es sich um das trimere Kondensationsprodukt des Acetons. Die Herstellung von Isophoron erfolgt über eine katalysierte Aldol-Kondensation von Aceton.

### Stand der Technik:

Sowohl die bestehende Patentliteratur als auch die wissenschaftlichen Veröffentlichungen zur Herstellung von Isophoron lassen sich im Wesentlichen in zwei Bereiche einteilen. Man unterscheidet zwischen Flüssigphasen- und Gasphasenprozessen. In CN 101633610A wird auch die Kondensationsreaktion zur Herstellung von Isophoron mit überkritischem Aceton beschrieben.

Während in den beschriebenen Gasphasenprozessen zum größten Teil mit heterogenen, festen Katalysatoren gearbeitet wird, kommen in Flüssigphasenprozessen sowohl homogene als auch heterogene Katalysatorsysteme zum Einsatz.

### a) Verfahren zur Herstellung von Isophoron in der Flüssigphase

Die Reaktion in der Flüssigphase wird in der Patentliteratur nahezu ausschließlich unter alkalischen Bedingungen bei erhöhten Temperaturen und hohen Drücken beschrieben.

Auf dem Gebiet der Isophoronchemie sind mehrere Patente der Shell Development Company bekannt (US 2,351,352, US 2,344,226, US 2,399,976, US 2,419,051). Unter anderem wird in der US 2,399,976 ein Kondensationsverfahren zur Herstellung von Isophoron im Kreislaufreaktor mittels Alkalikatalyse beschrieben. Die verwendete Lauge wird in dem Verfahren nach Phasentrennung wieder in den Reaktor zurückgeführt, während das entstandene Reaktionswasser mit der organischen Phase aus dem Reaktorkreislauf entfernt wird.

Des Weiteren ist in US 2,419,051 ein Verfahren beschrieben, in dem durch Hydrolyse der höheren Kondensationsprodukte ein Teil der Überkondensate zurückgespalten werden kann. Die Hydrolyse wird in einem Druckreaktor bei Temperaturen zwischen 130 - 235 °C mit einer erhöhten Laugekonzentration durchgeführt.

Um in der Synthese eine Phasentrennung zu verhindern, und somit eine einphasige Reaktionsführung zu erreichen, sind in den Anmeldungen der Societe Industrielle Des Derivatives De L'Acetylene (DE 10 58 047, GB733650) Alkohole als Lösungsvermittler beschrieben. Dieses Verfahren führt zu einer geringeren Reaktionszeit. Des Weiteren ist dort beschrieben, dass die Rückführung von abgetrennten Nebenprodukten in die Reaktionszone des Reaktors die Selektivität der Isophoronbildung steigert.

In den Patentdokumenten der Hibernia Chemie (DE 10 95 818, DE 11 44 269, DE 12 05 525, DE 11 65 018) aus den 1960er Jahren wird neben dem Einsatz eines einphasigen Edukt / Katalysator-Gemisches mit niedrigen Lauge-Konzentrationen auch die Aufarbeitung mittels Hydrolysekolonne beschrieben. Isophoron wird hier in einem Druckreaktor durch Kondensation von Aceton in flüssiger Phase mittels Alkalimengen (NaOH oder KOH) von weniger als 1 % als Katalysator und unter Anwendung von Wassermengen von unterhalb 20 % bei Temperaturen von 150 - 250 °C hergestellt. Die sich bei der Reaktion ausbildenden zwei Phasen werden sowohl durch eine geeignete Reaktionsführung (Reaktorbau, Impulsgenerator), als auch durch den Einsatz eines Emulgators emulgiert, um einen guten Kontakt zwischen Katalysator und den Reaktanten zu gewährleisten (DE 10 95 818).

Daneben wird in DE 12 05 525 die Aufarbeitung von Nebenprodukten, der sog. Überkondensate beschrieben. Bei 120 - 300 °C findet mit einer wässrigen Alkali-Lösung in einer sogenannten Druckdestillationskolonne unter laufender Entfernung des gebildeten Acetons die Hydrolyse der Überkondensate statt.

Die Gewinnung von reinem Isophoron aus Isophoron-haltigen Kondensationsprodukten gelingt durch eine Abtrennung der Leichtsieder durch Destillation unter demselben Druck, bei dem die Kondensation durchgeführt wird und durch eine weitere Aufarbeitung der noch bestehenden Überkondensate durch Destillation bei vermindertem Druck (DE 11 44 269).

Laut der Anmeldung von BP Chemicals lässt sich durch Verwendung von Kalilauge (KOH) anstelle des sonst üblichen Katalysators Natronlauge (NaOH) die Isophoronausbeute bei gleichbleibender Selektivität um bis zu 7 % steigern (DE 25 20 681).

Weiterhin ist beschrieben, dass die Produktqualität des Isophorons erhöht werden kann, indem man farbbildende Substanzen in einem Seitenstrom aus der Reaktionskolonne ausschleust, und diesen Strom durch Destillation und saure Umsetzung aufreinigt (DE 26 45 281).

Weiterhin existieren Anmeldungen zur Isophoron-Herstellung von Daicel Chemical Industries (JP 8245485, JP 8245486) aus den 1990er Jahren. Diese beschreiben, dass durch Erniedrigung der Wasserkonzentration im Eduktstrom als auch durch Rückführung der wässrigen Laugephase nach der Phasentrennung in den Hydrolyseteil der Reaktivdestillation der Isophoronumsatz gesteigert werden kann.

Neben den bisher genannten Flüssigphasenprozessen mittels homogener Katalysatorsysteme gibt es auch eine Patent-Veröffentlichung mit heterogenen Katalysatorsystemen in der Flüssigphase.

So beschreibt Elf Atochem S. A. in dem Patent US 5,849,957 die Verwendung von Hydrotalciten (Mg₁₋ₓAlₓO₁₊ₓ) als heterogenes Katalysatorsystem für die Herstellung von Isophoron. In diskontinuierlichen Rührkesselversuchen konnten mit einem solchen Katalysator ein Acetonumsatz von 38 % und eine Selektivität zum Isophoron von 51 % erreicht werden.

### b) Verfahren zur Herstellung von Isophoron in der Gasphase

Isophoron kann auch mittels heterogener Katalysatoren in der Gasphase hergestellt werden.

In den Dokumenten der Union Carbide (US 4,086,188, US 4,165,339, EP 095 783) wird die Herstellung von Isophoron mittels Lithium-, bzw. Zink-dotierter Hydrotalcit-artiger Fällungskatalysatoren beschrieben. Mit diesen Katalysatoren kann bei einem Acetonumsatz von 24 % eine Selektivität von 47 % bezüglich Isophoron erreicht werden (US 4,086,188) und der Katalysator durch Abbrennen der Verkokungsrückstände vollständig regeneriert werden (US 4,165,339). Durch Optimierung der Präparationsbedingungen kann die Standzeit eines solchen Katalysators auf bis zu ca. 1000 Stunden erhöht werden (EP 095 783).

In den Patenten der Aristech Chemical Corporation (WO9012645, WO9507255 sind verschiedene oxidische Magnesium/Aluminium-Katalysatoren beschrieben, die durch Aufschlämmung von Pseudoboehmit und Magnesiumoxid hergestellt werden (WO 9012645). Bei einem Aceton Umsatz von 30 % liegt die Selektivität bezüglich Isophoron bei 76 %. Neben den Katalysatoren beschreibt die Aristech Chemical Company auch ein Verfahren zur Herstellung von Isophoron in der Gasphase in einem Festbettreaktor (WO 95072559). Der Acetonumsatz wird hierbei auf 10 - 35 % begrenzt, um die Bildung von Verkokungsrückständen zu minimieren.

Weiterhin gibt es eine Reihe von Anmeldungen (JP 9059204, JP 9151152, JP 9151153, JP 9157207, JP 9157208, JP 9169687, JP 9169688) von Mitsui Toatsu Chemicals, die verschiedene Zeolith- und Magnesium/Alkalimetall-Katalysatoren für die Herstellung von Isophoron beanspruchen.

In wissenschaftliche Veröffentlichungen ist neben den bereits in den Patenten genannten Katalysatorsystemen ebenfalls die Verwendung von Kohlenstoff-Nanoröhrchen als Katalysator für die Isophoron-Synthese beschrieben. M. G. Stevens (Chem. Commun. 3, 1999) erreicht mit Cäsium-dotierten Kohlenstoff-Nanoröhrchen einen AcetonUmsatz von 11.9 % bei einer Isophoron-Selektivität von 61 %.

In der Patentanmeldung DE 102010062587.6 (CN 201010625116.6) ist ein Verfahren zur Herstellung von Isophoron wie folgt beschrieben:
Verfahren zur Herstellung von Isophoron durch katalysierte Aldol-Kondensationen von Aceton als Edukt,
Aufarbeitung des Reaktionsproduktes,
Hydrolyse des Wertstroms und Trennung in eine organische und eine wässrige Fraktion, Gewinnung von Isophoron aus der organischen Fraktion,
destillative Aufarbeitung der wässrigen Fraktion und Weiterleitung der Brüden vom Kopf der Apparatur der destillativen Aufarbeitung in die Hydrolyseapparatur.

Unter diesen Produktionsbedingungen kann es jedoch aufgrund der Eigenschaften der vorhandenen Stoffgemische zu Schaumbildungen insbesondere im Bereich der Abwasserkolonne, in der die destillative Aufarbeitung der wässrigen Fraktion stattfindet, kommen. Dieses kann zur Folge haben, dass Fehlmessungen in den elektronischen Mess- und Regeltechnik (EMR)-Einrichtungen auftreten und somit die Regelung des Prozesses erschwert ist. Insbesondere die Wärmeübertragung und / oder das Trennverhalten in der Abwasserkolonne sind dadurch schwer zu kontrollieren. Die Folge davon ist, dass nicht dauerhaft am ökonomischen und auch am ökologischen Optimum gearbeitet werden kann.

Bei der Synthese von Isophoron entsteht eine ganze Reihe von unerwünschten Nebenprodukten. Diese sind bspw. Diacetonalkohol, Mesityloxid, Phoron, Mesitylen sowie eine Reihe von höheren Kondensationsprodukten (Überkondensate) des Acetons (z. B. Xylitone und Isoxylitone). Aus diesem Grund ist die Erzielung von hohen Ausbeuten und Selektivitäten an Isophoron schwierig zu erreichen.

Die technische Aufgabe dieser Erfindung war es daher, ein Verfahren zu finden, das es ermöglicht, die Wirtschaftlichkeit der Isophoron Herstellung durch Vermeidung von Schaumbildung insbesondere im Bereich der Abwasserkolonne zu erhöhen. Dabei sollten auch ökologische Aspekte berücksichtigt sein.

Überraschend wurde gefunden, dass durch die Zugabe einer oberflächenaktiven Substanz - nachfolgend Entschäumer genannt - die Schaumbildung im Aufarbeitungsteil verhindert und bereits gebildeter Schaum zerstört wird und somit eine effiziente und sichere Fahrweise für die Isophoron-Herstellung ermöglicht wird.

Gegenstand der Erfindung ist ein Verfahren zur Herstellung von Isophoron durch
1. katalysierte Aldol-Kondensationen mit Aceton als Edukt,
2. Aufarbeitung des Reaktionsproduktes,
3. Hydrolyse des Wertstroms und Trennung in eine organische und eine wässrige Fraktion, Gewinnung von Isophoron aus der organischen Fraktion,
4. destillative Aufarbeitung der wässrigen Fraktion in Gegenwart mindestens eines Entschäumers und Weiterleitung der Brüden vom Kopf der Apparatur der destillativen Aufarbeitung in die Hydrolyse Apparatur
dadurch gekennzeichnet, dass als Entschäumer Polyetherpolyole eingesetzt werden.

Weiterhin ist Gegenstand der Erfindung ein Verfahren zur Herstellung von Isophoron wobei 5. das Wasser aus dem Sumpf der destillative Aufarbeitung der wässrigen Fraktion einer Entspannungsverdampfung unterworfen wird und das entstehende gereinigte Wasser in den Prozess zur Herstellung von Isophoron zurückgeführt wird.

Es wurde gefunden, dass durch die Zugabe eines Entschäumers in Stufe 4., insbesondere in den Zulauf der Abwasserkolonne, die Schaumbildung im Sumpf dieser Abwasserkolonne nahezu vollständig verhindert werden kann und somit eine sehr gute Trennung zwischen dem Hauptbestandteil Wasser und den noch gelösten organischen Komponenten, wie z.B. Isophoron, Aceton und andere Leichtsieder, sowie höhere siedende Produkte, erzielt werden kann. Außerdem lässt sich somit die Füllstandshöhe der Abwasserkolonne exakt bestimmen, und sowohl ein Überlaufen als auch ein Trockenlaufen der Abwasserkolonne kann verhindern werden.

Das erfinderische Verfahren kann kontinuierlich, diskontinuierlich oder semikontinuierlich durchgeführt werden. Es wird jedoch in bevorzugter Weise kontinuierlich durchgeführt.

### Stufe 1.

Die Herstellung von Isophoron erfolgt über katalysierte Aldol-Kondensationen mit Aceton als Edukt. Dabei reagieren im ersten Schritt zwei Aceton-Moleküle über das Zwischenprodukt Diacetonalkohol unter Wasserabspaltung zu Mesityloxid. In einer Folgereaktion reagiert das Mesityloxid mit einem weiteren Aceton wiederum unter Wasserabspaltung zum Isophoron.

Bei Isophoron handelt es sich also um das Reaktionsprodukt einer Kondensation von drei Molekülen Aceton unter der Abspaltung von zwei Molekülen Wasser.

Als Folge der chemischen Ähnlichkeit des eingesetzten Edukts (Aceton) und der gebildeten (Zwischen-) Produkte verläuft die Isophoron-Synthese nicht allzu selektiv. Aufgrund der Vielzahl von konkurrierenden Aldol-Kondensationsreaktionen erhält man unter Reaktionsbedingungen neben dem gewünschten Zielmolekül Isophoron sowohl eine ganze Reihe von unerwünschten (höheren) Kondensationsprodukten (z. B. Xylitone und Isoxylitone) als auch weitere Nebenkomponenten (z. B. Mesitylen).

Die Isophoron-Synthese ist also durch ein komplexes Reaktionsnetzwerk gekennzeichnet; die Selektivität ist in hohem Maße vom Umsatz abhängig. Um die Bildung von unerwünschten (höheren) Kondensationsprodukten zu minimieren, muss der Acetonumsatz begrenzt werden. Insbesondere in der Gasphasenreaktion kann der verwendete Katalysator durch sich bildende Verkokungsrückstände desaktiviert werden.

Es wurde gefunden, dass das entstehende Reaktionsgemisch durch das erfinderische Verfahren besonders wirtschaftlich und ökologisch zu Isophoron aufgearbeitet werden kann.

Die Kondensationsreaktion von Aceton zu Isophoron (Reaktion) wird bevorzugt in einer katalysierten Flüssigphasenreaktion durchgeführt. Alternativ lässt sich Isophoron auch mittels einer Gasphasenreaktion oder auch durch Reaktion in überkritischem Aceton herstellen.

Für die Durchführung der Reaktion gemäß dem erfindungsgemäßen Verfahren in der Flüssigphase wird das Aceton innerhalb des verwendeten Reaktors durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt, wobei die angegebenen Werte beliebig miteinander kombiniert werden können.

Für die Durchführung der Reaktion gemäß dem erfindungsgemäßen Verfahren in der Gasphase wird das Aceton innerhalb des verwendeten Reaktors durch katalytische Reaktion bei Temperaturen im Bereich von 100 bis 400 °C, bevorzugt 200 - 400 °C zu Isophoron umgesetzt.

Für die Durchführung der Reaktion gemäß dem erfindungsgemäßen Verfahren im überkritischen Bereich wird das Aceton innerhalb des verwendeten Reaktors durch katalytische Reaktion bei Temperaturen im Bereich von 250 bis 350 °C und einem Druckbereich von 50 bis 200 bar zu Isophoron umgesetzt.

Die katalytische Reaktion kann mit den im Stand der Technik genannten Katalysatoren durchgeführt werden, dabei kann es sich entweder um einen homogenen oder einen heterogenen Katalysator handeln. In der Flüssigphase wird bevorzugt ein homogener Katalysator eingesetzt, in der Gasphase bevorzugt ein heterogener Katalysator verwendet. Für die Reaktion im überkritischen Bereich können sowohl homogene als auch heterogene Katalysatoren verwendet werden.

Bei der bevorzugten Reaktion in der Flüssigphase lässt sich Isophoron mittels homogenem Katalysator mit Alkalimengen (NaOH oder KOH) von < 1 Gew.-%, bevorzugt von < 0,5 Gew.-%, besonders bevorzugt < 0,2 Gew.-%, herstellen. Besonders bevorzugt wird als Katalysators NaOH in Mengen von 0,015 bis 0,05 Gew.-% eingesetzt. Die eingesetzte Wasserkonzentration ergibt sich u. a. aus den Rückführströmen der Aufarbeitungsprozesse, sie sollte bezogen auf die gesamte Flüssigkeitsmenge unterhalb < 40 %, bevorzugt < 30 % liegen.

Die Reaktion kann in beliebigen Reaktoren nach dem Stand der Technik, wie z. B. Rohrreaktoren, Rührkesseln, Rührkesselkaskaden, Festbettreaktoren, Druckdestillationsreaktoren bzw. Reaktivdestillationen, mikrostrukturierten Reaktoren, Schlaufenreaktoren usw. oder in Kombinationen aus beliebigen Reaktoren durchgeführt werden. Dabei ist die Wahl der Reaktoren nicht auf die genannte Auswahl beschränkt.

Der Begriff Druckdestillationsreaktor ist hier gleichzusetzen mit Apparaten, in denen eine Reaktivdestillation durchgeführt wird. Die Reaktivdestillation ist in der Fachliteratur hinlänglich beschrieben, z. B. in Ullmann's Encylcopedia of Industrial Chemistry (M. Sakuth, D. Reusch, R. Janowsky: Reactive Distillation © 2008 Wiley-VCH Verlag GmbH & Co KGaA, Weinheim, DOI: 10.1002/14356007.c22_c01.pub2). Hier und in der zitierten Literatur sind alle gängigen Prozesse und Apparate der Reaktivdestillation beschrieben. Wird im folgenden Text der Patentschrift der Begriff der Reaktivdestillationskolonne verwendet, so sind sämtliche Ausführungsförmen der Reaktivdestillation, wie in der Literatur beschrieben, gemeint.

In bevorzugter Ausführung erfolgt die Reaktionsdurchführung in Reaktivdestillationskolonnen, Rohrreaktoren oder Festbettreaktoren. Besonders bevorzugt sind Rohrreaktoren.

### Stufe 2.

Nach Durchführung der Reaktion wird das Reaktionsgemisch aufgearbeitet (Aufarbeitung) und in die einzelnen Komponenten getrennt. Diese sind neben Isophoron sogenannte Leichtsieder wie z. B. Aceton, Diacetonalkohol und Mesityloxid, sowie eine Reihe von höheren Kondensationsprodukten (Überkondensate) des Acetons (z. B. Xylitone und Isoxylitone), Wasser und gegebenenfalls Katalysator. Dabei wird die Trennung ganz oder teilweise durchgeführt.

Die Abtrennung der einzelnen Fraktionen kann mit allen Trennmethoden wie z. B. Destillation, Entspannungsverdampfung, Kristallisation, Extraktion, Sorption, Permeation, Phasentrennung oder Kombinationen aus den genannten, kontinuierlich oder absatzweise, ein- oder mehrstufig durchgeführt werden. Bevorzugt wird die Trennung durch Destillation in einem oder mehreren Apparaten erreicht.
Dabei kann die Destillation räumlich getrennt von der Isophoronsynthese (Reaktion) durchgeführt werden oder in einem Apparat stattfinden. Bevorzugt erfolgt die Abtrennung der einzelnen Fraktionen durch eine Reaktivdestillation, bevorzugt in einer Reaktivdestillationskolonne.

Besonders bevorzugt wird die Abtrennung räumlich getrennt von der Isophoronsynthese (Reaktion) in einer Reaktivdestillationskolonne mit einer Seitenstromentnahme durchgeführt.

Der so erhaltene Wertstoffstrom, insbesondere Isophoron, höherkondensierten Produkten und Wasser und gegebenenfalls Katalysator, wird in einer 3. Stufe weiterverarbeitet, wie weiter unten beschrieben.

Bevorzugt erfolgt die Abtrennung in Stufe 2. in drei Fraktionen:
a) Einer Fraktion aus nicht umgesetzten Aceton, Wasser und Leichtsiedern, wie z. B. Diacetonalkohol und Mesityloxid, die kondensiert und anschließend zur Reaktion in den Reaktor zurückgeführt wird.
b) Einer Fraktion, in der insbesondere farbgebende Substanzen angereichert werden. Diese Fraktion wird weiter aufgereinigt und die enthaltenden Wertstoffe werden im Prozess zurückgeführt.
c) Einer Fraktion aus insbesondere Isophoron, höherkondensierten Produkten und Wasser und gegebenenfalls Katalysator, genannt Wertstrom. Diese Fraktion wird anschließend einer Hydrolyse unterworfen.

Die Fraktion a) wird in der bevorzugten Ausführungsform als Brüdenstrom entnommen, enthaltend im Wesentlichen Aceton, Wasser und Leichtsieder, im Wesentlichen Diacetonalkohol und Mesityloxid, kondensiert und dem Reaktor mit den Einsatzstoffen Aceton, Wasser und gegebenenfalls Katalysator wieder zugesetzt.
Die Fraktion b) wird in der bevorzugten Ausführungsform als Seitenstrom der Destillationskolonne, bevorzugt einer Reaktivdestillationskolonne, entnommen, gegebenenfalls neutralisiert und weiter aufgearbeitet. Dabei können bei der Aufarbeitung alle gängigen Trennmethoden wie z. B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig durchgeführt werden. Bevorzugt wird die Aufreinigung durch Destillation erreicht. Besonders bevorzugt wird die Aufreinigung durch eine Kombination aus Neutralisation oder Extraktion und anschließender Destillation, bevorzugt in einer Reaktivdestillationskolonne, erreicht. Bevorzugt wird die aufgearbeitete Phase mit den Wertprodukten aus Isophoron, Hochsiedern und gegebenenfalls Katalysator in die Hydrolyse geführt. Eine weitere erhaltene Phase aus Wertprodukten, im Wesentlichen enthaltend Aceton, Diacetonalkohol und Mesityloxid, wird bevorzugt in die Reaktion zurückgeführt. Gegebenenfalls anfallende Rückstände werden der thermischen Verwertung zugeführt.

### Stufe 3.

Der Wertstoffstrom aus Stufe 2, bevorzugt die Fraktion c), wird einer Hydrolyse unterworfen. Das Ziel der Hydrolyse ist es, Nebenprodukte teilweise oder vollständig in Isophoron, Aceton und andere Wertprodukte zu überführen. Die Hydrolyse kann in allen gängigen Reaktoren, welche bereits oben beschrieben wurden, oder Destillationskolonnen oder Kombinationen aus beiden, durchgeführt werden. Bevorzugt wird die Hydrolyse durch eine Reaktivdestillation durchgeführt, in dem die entstehenden Leichtsieder, im Wesentlichen enthaltend Aceton, Diacetonalkohol und Mesityloxid, direkt aus der Hydrolysezone entfernt und in die Reaktion zurückgeführt werden, und somit nicht mehr für Nebenreaktionen in der Hydrolyse zur Verfügung stehen.

Ganz besonders bevorzugt wird die Hydrolyse der Fraktion c) in einem Apparat, durch eine Reaktivdestillation durchgeführt, bevorzugt in einer Reaktivdestillationskolonne, wobei gleichzeitig die Trennung des Reaktionsgemisches in die Fraktionen a) bis c) erfolgt, so dass die entstehenden Produkte gleichzeitig entsprechend aufgetrennt werden und die Fraktion c) hydrolysiert wird.

Gegebenenfalls können die Hydrolyse der Stufe 3. und die destillative Abtrennung der Stufe 2. auch in einem Apparat mit der Isophoronsynthese (Reaktion) stattfinden.

Die Hydrolyse kann in allen Mischungsverhältnissen der organischen Komponenten mit Wasser mit oder ohne Katalysator durchgeführt werden. Die Wasserkonzentration in der Hydrolyse beträgt dabei 0,1 - 99,9 Gew.-%, bevorzugt 30 - 90 Gew.-%. Im Falle der homogenen Katalyse wird bei der Hydrolyse bevorzugt derjenige Katalysator eingesetzt, der auch im Reaktionsteil verwendet wird. Bevorzugt werden Katalysatorkonzentrationen von 0,001 - 10 Gew.-%, besonders bevorzugt von 0,05 - 1 Gew.-%. Der Druck im Hydrolysereaktor beträgt 1-200 bar, bevorzugt 20 - 60 bar, besonders bevorzugt wird die Hydrolyse mindestens bei dem Druck durchgeführt, der auch im Isophoronsyntheseschritt (Reaktion) herrscht. Die Temperatur der Hydrolyse beträgt 100 - 300 °C, bevorzugt 210 - 260 °C. Besonders bevorzugt wird sich bei Verwendung einer Reaktivdestillationskolonne eine Temperatur bzw. ein Temperaturverlauf einstellen, der den Siedetemperaturen im Sumpf und auf den einzelnen Trenn- oder Reaktionsstufen entspricht.

Die Hydrolyse kann in einem oder mehreren Apparaten einstufig oder mehrstufig durchgeführt werden.

Die so aufgearbeitete Fraktion des Wertstoffstroms, bevorzugt die Fraktion c) wird anschließend aus dem Hydrolysereaktor bzw. der Reaktivdestillationskolonne abgetrennt, abgekühlt und einer Phasentrennung (Trennung) unterworfen.

Die Phasentrennung in Stufe 3. erfolgt in eine im Wesentlichen organische Fraktion, bevorzugt Fraktion d) und eine im Wesentlichen wässrige Fraktion, bevorzugt Fraktion e), die, bei homogener Katalyse, auch den Katalysator enthält. Dabei können übliche Phasentrennbehälter mit und ohne Einbauten zum Einsatz kommen. Die Phasentrennung erfolgt bei einer Temperatur zwischen 0 - 200 °C, bevorzugt bei 0 - 100 °C, besonders bevorzugt bei 20 - 70 °C und einem Druck von 1 - 150 bar, bevorzugt 20 - 60 bar, besonders bevorzugt bei dem Druck, der auch in der Hydrolyse herrscht.

Die im Wesentlichen organische Fraktion der Stufe 3., bevorzugt Fraktion d), mit dem Zielprodukt Isophoron wird gegebenenfalls neutralisiert und mit üblichen Methoden aufgereinigt, so dass ein Isophoron mit der gewünschten Reinheit und Farbstabilität erhalten wird (Gewinnung). Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig, unter Druck oder im Vakuum durchgeführt werden. Bevorzugt wird die Aufreinigung durch Destillation erreicht. Besonders bevorzugt wird die Aufreinigung durch eine Kombination aus Neutralisation oder Extraktion und folgender Destillation erreicht.

### Stufe 4.

An dieser Stelle wird die destillative Aufarbeitung der wässrigen Fraktion aus Stufe 3., bevorzugt Fraktion e), (Abwasseraufreinigung) und Weiterleitung der Brüden vom Kopf der Apparatur der destillativen Aufbereitung in die Hydrolyseapparatur genauer beschrieben.

Die im Wesentlichen wässrige Fraktion aus Stufe 3., bevorzugt Fraktion e), wird einer Abwasseraufreinigung zugeführt. Hier erfolgt die Trennung des Reaktionswassers als Hauptbestandteil und gegebenenfalls des Katalysators von gegebenenfalls noch gelösten organischen Komponenten, wie z. B. Isophoron, Aceton und höher kondensierten Produkten.

Die Abwasseraufreinigung wird bevorzugt in einer oder mehreren Destillationskolonnen durchgeführt, auch hier als Abwasserkolonnen bezeichnet.

Diese Stufe 4. wird erfindungsgemäß in Gegenwart mindestens eines Entschäumers durchgeführt, wodurch die Schaumbildung im Sumpf dieser Abwasserkolonne nahezu vollständig verhindert wird und somit eine sehr gute Trennung zwischen dem Hauptbestandteil Wasser und den noch gelösten organischen Komponenten, wie z. B. Isophoron, Aceton und andere Leichtsieder, sowie höhere siedende Produkte, erzielt wird. Außerdem lässt sich somit die Füllstandshöhe der Abwasserkolonne exakt bestimmen, und sowohl ein Überlaufen als auch ein Trockenlaufen der Abwasserkolonne wird verhindert.

Insbesondere erfolgt die Zugabe des Entschäumers in den Zulauf der Abwasserkolonne.

Bei den erfindungsgemäß eingesetzten Entschäumern handelt es sich um chemische Substanzen zur Schaumbekämpfung ausgewählt aus Polyetherpolyolen. Besonders bevorzugt handelt es sich dabei um eine wässrige Mischung mindestens eines Polyetherpolyols.

Im Allgemeinen sind 0,00005 bis 0,5 Gew.-% Entschäumer bezogen auf die chemische Substanz, also auf den Entschäumer, in der Abwasserkolonne enthalten. Die Gesamtmenge an Entschäumer in der Abwasserkolonne beträgt bevorzugt <0,1 Gew.-%, besonders bevorzugt < 0,01 Gew.-%, bezogen auf die chemische Substanz, also auf den Entschäumer.

Bevorzugt wird der Entschäumer so in den Zulauf der Abwasserkolonne gegeben, dass die Gesamtmenge an Entschäumer < 0,5 Gew.-%, bevorzugt < 0,1 Gew.-%, besonders bevorzugt < 0,01 Gew.-%, in der Abwasserkolonne beträgt, bezogen auf die chemische Substanz, also auf den Entschäumer.

Durch die Zugabe des Entschäumers werden mehrere Probleme des derzeitigen Standes der Technik gelöst:
1.) Durch die Zugabe eines Entschäumers, bevorzugt in den Zulauf der Abwasserkolonne, wird die Schaumbildung im Sumpf der Abwasserkolonne nahezu vollständig verhindert.
2.) Es lässt sich eine bessere Trennung zwischen dem Hauptbestandteil Wasser und den noch vorhandenen organischen Komponenten, z. B. Isophoron, Aceton sowie andere Leichtsieder und höher kondensierte Produkte erreichen, und somit eine höhere Produktausbeute erzielen.
3.) Die Regelbarkeit der Anlage, insbesondere der Abwasserkolonne wird erhöht, da durch die Verwendung des Entschäumers eine konstante Fahrweise gegeben ist.

Die Abwasseraufreinigung wird bevorzugt in einer oder mehreren Destillationskolonnen (Abwasserkolonnen) durchgeführt. Die Brüden der Abwasserkolonne werden direkt in den Apparat geleitet, in dem die Hydrolyse stattfindet. Da die Brüden im Wesentlichen aus Wasser bestehen, stellt sich im Hydrolyseteil somit eine notwendige, ausreichend hohe Wasserkonzentration ein, so dass kein zusätzliches Frischwasser in die Hydrolyse eingebracht werden muss. Außerdem werden die in der Fraktion e) gelösten, organischen Anteile teilweise oder komplett über die Brüden der Abwasserkolonne in den Prozess zurückgeführt. Das minimiert die organische Belastung im Abwasser und, da es sich im Wesentlichen um Isophoron handelt, erhöht die Gesamtausbeute im Prozess. Diese Verschaltung der Abwasserkolonne trägt somit einen wesentlichen Beitrag zur ökologischen und ökonomischen Prozessführung bei. Desweiteren wird die notwendige Wärme für die Hydrolyse bzw. die destillative Trennung des Reaktionsgemisches durch die Brüden zur Verfügung gestellt, es wird keine separate Heizung benötigt.

Der Druck in der Abwasserkolonne beträgt 1 - 200 bar, bevorzugt 20 - 60 bar. Besonders bevorzugt wird bei dem Systemdruck gearbeitet, der sich im Gesamtsystem Hydrolyse/Abwasserkolonne einstellt, wenn die Brüden der Abwasserkolonne direkt in den Hydrolyseteil der Reaktivdestillation geleitet werden. Die Temperatur in der Abwasserkolonne entspricht der Siedetemperatur der Fraktion e) unter den Druckbedingungen. Die bevorzugte Temperatur der Brüden beträgt 200 - 300 °C.

### Stufe 5.

Im Folgenden wird näher beschrieben, wie das Wasser aus dem Sumpf der destillativen Aufarbeitung der wässrigen Fraktion einer Entspannungsverdampfung unterworfen wird und das entstehende gereinigte Wasser in den Prozess zur Herstellung von Isophoron zurückgeführt wird.

Das im Sumpf der Abwasserkolonne anfallende Abwasser (Strom f) kann abgekühlt und verworfen werden. Bevorzugt wird das Abwasser f) aber einer Entspannungsverdampfung zugeführt und somit weiter aufgetrennt. Die Brüden g) der Entspannungverdampfungsstufe, die im Wesentlichen aus reinem Wasser bestehen, können kondensiert und als Wasser in den Prozess, bevorzugt in die Reaktion, z. B. zur Verdünnung des eingesetzten Katalysators (im Falle der homogenen Katalyse) zurückgeführt werden. Dadurch wird die Abwassermenge nochmals reduziert. Die Entspannungsverdampfung kann ein- oder mehrstufig kontinuierlich oder diskontinuierlich durchgeführt werden. Der Druck in der Enspannungsverdampfung liegt in jedem Fall unterhalb des Druckes in der Abwasserkolonne. Bevorzugt ist bei dem erfindungsgemäßen Verfahren die Anwendung einer Enspannungsverdampfung.

Sämtliche Destillations- und Reaktionsschritte im Prozess können in Reaktoren oder Apparaten mit oder ohne Einbauten durchgeführt werden, wie z. B. Dephlegmatoren, ungeordnete Einbauten oder Schüttungen, geordnete Einbauten oder Packungen, Böden mit oder ohne Zwangsführung.

Alle für die Reaktion eingesetzten produktberührten metallischen Werkstoffe und die aus den metallischen Werkstoffen hergestellten Apparate sowie deren Einbauten müssen gegen Laugen beständig sein. Dabei können in Abhängigkeit von der Gefährdung unterschiedliche Beständigkeitsanforderungen bestehen. Für die Beständigkeiten sind nicht nur die chemischen und/oder mechanischen Eigenschaften von Bedeutung, sondern auch die zur Anwendung kommenden Fertigungsmethoden und die Beurteilungsmaßstäbe während der Prüfung.

Für die metallischen Werkstoffe wird zum Teil Bezug auf das AD 2000-Merkblatt HP 0 Ausgabe 11.2008 (Allgemeine Grundsätze für Auslegung, Herstellung und damit verbundene Prüfungen) und DIN EN 10020 Ausgabe 07.2000 (Begriffsbestimmung für die Einteilung der Stähle) genommen. Die dort genannten Werkstoffgruppen werden zitiert, um die Bezeichnungen (z. B. "austenitscher nichtrostender Stahl) zu präzisieren. Sofern technisch sinnvoll, gelten die Aussagen für alle technisch verfügbaren Varianten der Werkstoffe (z. B. Schmiedevarianten, Walzvarianten und Gussvarianten) mit vergleichbarer Beständigkeit gegen Laugekorrosion.
a) Für drucktragende und produktberührte Komponenten können beliebige, nach dem Stand der Technik geeignete Werkstoffe zur Anwendung kommen wie z. B.:
   - Warmfeste Stähle (z. B. Werkstoffuntergruppe 5.1 bis 5.4 und 6.1 bis 6.4 gemäß AD 2000 HP 0)
   - Austenitische nichtrostende Stähle (z. B. Werkstoffuntergruppe 8.1 bis 8.2 gemäß AD 2000 HP 0)
   - Ferritfreie austenitische nichtrostende Stähle (z. B. Werkstoffuntergruppe 8.1 bis 8.2 gemäß AD 2000 HP 0)
   - Ferritisch-austenitische nichtrostende Stähle (z. B. Werkstoffuntergruppe 10.1 bis 10.2 gemäß AD 2000 HP 0)
   - Nickel und Nickellegierungen (z. B. Werkstoffuntergruppe 41 bis 46 gemäß AD 2000 HP 0)

Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine technische Beständigkeit gegen Laugen auszeichnen. Ein Verzicht auf Wärmebehandlungen ist nicht möglich, wenn sich dadurch die technische Beständigkeit gegen Laugen in unzulässiger Weise ändert.
b) Für nicht drucktragende und produktberührte Komponenten können beliebige, nach dem Stand der Technik geeignete Werkstoffe zur Anwendung kommen wie z. B.:
   - Alle unter a) genannten Werkstoffe
   - Unlegierte Stähle (z. B. Werkstoffuntergruppe 1.1 bis 1.2 gemäß AD 2000 HP 0)
   - Unlegierte Stähle und andere legierte Stähle (z. B. gemäß DIN EN 10020)

Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine ausreichende Beständigkeit gegen Laugen auszeichnen. Für nicht drucktragende Komponenten können in Abhängigkeit von der Gefährdung gegebenenfalls temporäre Beständigkeiten in Kauf genommen werden. Ein Verzicht auf Wärmebehandlungen ist nicht möglich, wenn sich dadurch die technische Beständigkeit gegen Laugen in unzulässiger Weise ändert.
c) Die Werkstoffeigenschaften werden durch geeignete Fertigungsverfahren verändert, die im Folgenden nach den in der DIN 8580 Ausgabe 09.2003 (Fertigungsverfahren - Begriffe, Einteilung) genannten Bezeichnungen beschrieben sind. Folgende Fertigungsverfahren können für die Verarbeitung der metallischen Werkstoffe z. B. zur Anwendung kommen:
   - Urformen (z. B. Gießen)
   - Umformen (z. B. Kaltumformen und Warmumformen)
   - Trennen (z. B. Spanen mit geometrisch bestimmter Schneide und Spanen mit geometrisch unbestimmter Schneide)
   - Fügen (z. B. Schmelzschweißen)
   - Beschichten (z. B. Beschichten aus dem flüssigen Zustand, Schmelztauchen, Plattieren, Thermisches Spritzen, Sintern, galvanisches Besichten, chemisches Beschichten und Beschichten aus dem gasförmigen und dampfförmigen Zustand)
   - Stoffeigenschaften ändernde Fertigungsverfahren (Verfestigung durch Umformen wie z. B. Schmieden, Walzen, Strahlen; Wärmebehandeln wie z. B. Vergüten, Rekristallisationsglühen, Spannungsarmglühen, Normalisierungsglühen; Thermomechanische Behandlungen wie z. B. die Kombination von Wärmebehandlung und Umformbehandlung; Sintern und Brennen)

Auch Kombinationen der o. g. Fertigungsverfahren können zur Anwendung kommen. Dabei ist die Wahl der Fertigungsverfahren nicht auf die genannte Auswahl beschränkt. Bevorzugt werden Verfahren, die nach dem Stand der Technik die erforderliche Laugenbeständigkeit der jeweiligen Werkstoffe und Apparate gewährleisten.
d) Folgende Prüfungen an Apparaten und Einbauten sowie insbesondere an deren Schweißverbindungen können beispielsweise zur Anwendung kommen:
   - Magnetpulverprüfung MT
   - Eindringprüfung PT
   - Durchstrahlungsprüfung RT
   - Ultraschallprüfung UT
   - Sichtprüfung VT
   - Härteprüfung HT
   - Legierungsanalyse

Auch Kombinationen der o. g. Prüfverfahren sind möglich. Dabei ist die Wahl der Prüfverfahren nicht auf die genannte Auswahl beschränkt. Es werden Prüfverfahren und Bewertungsgrundlagen bevorzugt, die nach dem Stand der Technik dazu beitragen, die erforderliche Laugenbeständigkeit der jeweiligen Komponenten zu gewährleisten.

### Vergleichsbeispiel, nicht erfindungsgemäß:

Eine im Wesentlichen wässrige Lösung mit einem organischen Anteil (Isophoron, Aceton sowie andere Leichtsieder und höher kondensierte Produkte) von < 5 Gew.-% wird destillativ aufgearbeitet um den organischen Anteil von der wässrigen Phase zu trennen. Die Temperatur der Brüden liegt dabei im Bereich zwischen 200 und 300 °C und der Druck in der Abwasserkolonne im Bereich von 20 - 60 bar. Die Brüden vom Kopf der Abwasserkolonne werden dabei einer Hydrolysekolonne zugeführt. Innerhalb von ca. 5 Stunden kommt es dabei in der Abwasserkolonne zu einer starken Schaumbildung, die eine reproduzierbare Messung der Füllstandhöhe verhindert und somit auch eine konstante Fahrweise unmöglich macht. Nur durch manuelles permanentes Eingreifen unter Zuhilfenahme von Hilfsgrößen (1. Temperaturmessung im Sumpf der Kolonne und 2. Differentdruckmessung), und somit einem deutlich erhöhten Regelaufwand, ist es möglich die Kolonne weiterhin zu betreiben. Die Folge davon ist, dass die Trennung nicht dauerhaft optimal erfolgen kann. Im Extremfall kann es dazu kommen, dass die Abwasserkolonne trocken läuft (=> Erschwerung des Wärmeübertrags), bzw. dass die Kolonne überläuft und somit die Hydrolyse in der Hydrolysekolonne nicht optimal durchgeführt werden kann. Daher kann mit einer solchen Fahrweise nicht die optimale Ausbeute erzielt und nicht dauerhaft am ökonomischen und ökologischen Optimum gearbeitet werden.

### Beispiel, erfindungsgemäß:

Eine im Wesentlichen wässrige Lösung mit einem organischen Anteil (Isophoron, Aceton sowie andere Leichtsieder und höher kondensierte Produkte) von < 5 Gew.-% wird destillativ aufgearbeitet um den organischen Anteil von der wässrigen Phase zu trennen. Die Temperatur der Brüden liegt dabei im Bereich zwischen 200 und 300 °C und der Druck in der Kolonne (Abwasserkolonne) im Bereich von 20 - 60 bar. Die Brüden vom Kopf der Abwasserkolonne werden dabei einer Hydrolysekolonne zugeführt. Der Abwasserkolonne wird eine wässrige Lösung eines Polyetherpolyol als Entschäumer hinzugefügt, so dass die Gesamtmenge an Polyetherpolyol in der Abwasserkolonne: 0,01 Gew.-% beträgt. Durch die Zugabe des Polyetherpolyols wird die Schaumbildung nahezu vollständig verhindert und somit kann die Abwasserkolonne mittels der Füllstandsmessung automatisch betrieben werden, ein manuelles Eingreifen ist nicht notwendig. Das Trennverhalten in der Abwasserkolonne ist somit optimal und es kann dauerhaft am ökonomischen und ökologischen Optimum gearbeitet werden.

Vergleich mit dem nicht erfindungsgemäßen Verfahren gemäß Vergleichsbeispiel: ca. 1 - 2 Gew.-% weniger Organikverlust über das Abwasser. Bezogen auf 1 Tonne Isophoronproduktion bedeutet das ca. 10-20 kg Isophoron Mehrproduktion und eine Minimierung der organischen Belastung des Abwassers.

## Patentansprüche

1. Verfahren zur Herstellung von Isophoron durch
1. katalysierte Aldol-Kondensationen mit Aceton als Edukt,
2. Aufarbeitung des Reaktionsproduktes,
3. Hydrolyse des Wertstroms und Trennung in eine organische und eine wässrige Fraktion,
Gewinnung von Isophoron aus der organischen Fraktion,
4. destillative Aufarbeitung der wässrigen Fraktion in Gegenwart mindestens eines Entschäumers und Weiterleitung der Brüden vom Kopf der Apparatur der destillativen Aufarbeitung in die Hydrolyse Apparatur,
**dadurch gekennzeichnet, dass** als Entschäumer Polyetherpolyole eingesetzt werden.

2. Verfahren zur Herstellung von Isophoron nach Anspruch 1, **dadurch gekennzeichnet, dass**
5. das Wasser aus dem Sumpf der destillativen Aufarbeitung der wässrigen Fraktion einer Entspannungsverdampfung unterworfen wird und das entstehende gereinigte Wasser in den Prozess zur Herstellung von Isophoron zurückgeführt wird.

3. Verfahren zur Herstellung von Isophoron nach mindestens einem der vorherigen Ansprüche, **dadurch gekennzeichnet, dass** als Entschäumer Mineralöle, natürliche Öle, höhere Alkohole, Polyole, Polyetherpolyole, Silikone, oder beliebige Mischungen der genannten Komponenten, eingesetzt werden.

4. Verfahren zur Herstellung von Isophoron nach **dadurch gekennzeichnet, dass** eine wässrige Mischung mindestens eines Entschäumers eingesetzt wird.

5. Verfahren zur Herstellung von Isophoron nach mindestens einem der vorherigen Ansprüche **dadurch gekennzeichnet, dass** eine wässrige Mischung mindestens eines Polyetherpolyols eingesetzt wird.

6. Verfahren zur Herstellung von Isophoron nach mindestens einem der vorherigen Ansprüche **dadurch gekennzeichnet, dass** 0,00005 bis 0,5 Gew.-% Entschäumer, bezogen auf die chemische Substanz, also auf den Entschäumer, in der Abwasserkolonne der Stufe 4. enthalten sind, bevorzugt < 0,1 Gew.-%, besonders bevorzugt < 0,01 Gew.-%.

7. Verfahren zur Herstellung von Isophoron nach mindestens einem der vorherigen Ansprüche **dadurch gekennzeichnet, dass** durch die Zugabe mindestens eines Entschäumers in den Zulauf einer Abwasserkolonne der destillativen Aufarbeitung der wässrigen Fraktion erfolgt.

8. Verfahren zur Herstellung von Isophoron nach mindestens einem der vorherigen Ansprüche, **dadurch gekennzeichnet, dass** der Entschäumer so in den Zulauf der Abwasserkolonne gegeben, dass die Gesamtmenge an Entschäumer < 0,5 Gew.-%, bevorzugt < 0,1 Gew.-%, besonders bevorzugt < 0,01 Gew.-% in der Abwasserkolonne beträgt, bezogen auf die chemische Substanz, also auf den Entschäumer.

9. Verfahren zur Herstellung von Isophoron nach mindestens einem der vorherigen Ansprüche 1- 8, **dadurch gekennzeichnet, dass** die Durchführung der Reaktion in Stufe 1. in der Flüssigphase erfolgt, wobei das Aceton durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt wird, wobei die angegebenen Werte beliebig miteinander kombiniert werden können.

10. Verfahren zur Herstellung von Isophoron nach mindestens einem der vorherigen Ansprüche 1- 8, **dadurch gekennzeichnet, dass** die Durchführung der Reaktion in Stufe 1. in der Gasphase erfolgt, wobei das Aceton durch katalytische Reaktion bei Temperaturen im Bereich von 100 bis 400 °C, bevorzugt 200 - 400 °C zu Isophoron umgesetzt wird.

11. Verfahren zur Herstellung von Isophoron nach mindestens einem der vorherigen Ansprüche 1- 8, **dadurch gekennzeichnet, dass** die Durchführung der Reaktion in Stufe 1. im Überkritischen Bereich erfolgt, wobei das Aceton durch katalytische Reaktion bei Temperaturen im Bereich von 250 bis 350 °C und einem Druckbereich von 50 bis 200 bar zu Isophoron umgesetzt wird.

12. Verfahren zur Herstellung von Isophoron nach mindestens einem der vorherigen Ansprüche, **dadurch gekennzeichnet, dass** in Stufe 1. ein homogener oder ein heterogener Katalysator, bevorzugt ein homogener Katalysator eingesetzt wird.

13. Verfahren zur Herstellung von Isophoron nach Anspruch 9, **dadurch gekennzeichnet, dass** die Reaktion in der Flüssigphase mit einem homogenen Katalysator erfolgt.

14. Verfahren zur Herstellung von Isophoron nach Anspruch 13, **dadurch gekennzeichnet, dass** Alkalimengen von < 1 Gew.-%, bevorzugt von < 0,2 Gew.-% als Katalysator, bevorzugt NaOH in Mengen von 0,015 bis 0,05 Gew.-%, eingesetzt werden.

15. Verfahren zur Herstellung von Isophoron nach mindestens einem der vorherigen Ansprüche, **dadurch gekennzeichnet, dass** die Reaktion in Stufe 1. in Reaktoren ausgewählt aus Rohrreaktoren, Rührkesseln, Rührkesselkaskaden, Festbettreaktoren, Reaktivdestillationskolonnen, mikrostrukturierten Reaktoren, Schlaufenreaktoren oder in Kombinationen aus diesen Reaktoren, durchgeführt wird.

16. Verfahren zur Herstellung von Isophoron nach mindestens einem der vorherigen Ansprüche, **dadurch gekennzeichnet, dass** die Reaktion in Stufe 1. in Reaktivdestillationskolonnen, Rohrreaktoren oder Festbettreaktoren, besonders bevorzugt in einem Rohrreaktor, durchgeführt wird.

17. Verfahren zur Herstellung von Isophoron nach mindestens einem der vorherigen Ansprüche, **dadurch gekennzeichnet, dass** nach Durchführung der Reaktion das Reaktionsgemisch in Stufe 2. aufgearbeitet und in die einzelnen Komponenten getrennt wird, wobei die Abtrennung ganz oder teilweise durchgeführt wird.

18. Verfahren zur Herstellung von Isophoron nach mindestens einem der vorherigen Ansprüche, **dadurch gekennzeichnet, dass** in Stufe 2. die Abtrennung der einzelnen Fraktionen durch Trennmethoden wie Destillation, Entspannungsverdampfung, Kristallisation, Extraktion, Sorption, Permeation, Phasentrennung oder Kombinationen aus den genannten, kontinuierlich oder absatzweise, ein- oder mehrstufig durchgeführt wird, bevorzugt durch Destillation in einem oder mehreren Apparaten.

19. Verfahren zur Herstellung von Isophoron nach mindestens einem der vorherigen Ansprüche, **dadurch gekennzeichnet, dass** in Stufe 2. die Destillation räumlich getrennt von der Isophoronsynthese durchgeführt wird oder in einem Reaktionsapparat stattfindet, bevorzugt durch eine Reaktivdestillation, bevorzugt in einer Reaktivdestillationskolonne.

20. Verfahren zur Herstellung von Isophoron nach mindestens einem der vorherigen Ansprüche, **dadurch gekennzeichnet, dass** in Stufe 2. die Abtrennung räumlich getrennt von der Isophoronsynthese durch eine Reaktivdestillation, bevorzugt in einer Reaktivdestillationskolonne, mit einer Seitenstromentnahme durchgeführt wird.

21. Verfahren zur Herstellung von Isophoron nach mindestens einem der vorherigen Ansprüche, **dadurch gekennzeichnet, dass** in Stufe 2. die Abtrennung in drei Fraktionen erfolgt:
a) Einer Fraktion aus nicht umgesetzten Aceton, Wasser und Leichtsiedern, die kondensiert und anschließend zur Reaktion in den Reaktor zurückgeführt wird;
b) Einer Fraktion, in der insbesondere farbgebende Substanzen angereichert werden, wobei diese Fraktion weiter aufgereinigt wird und die enthaltenden Wertstoffe im Prozess zurückgeführt werden;
c) Einer Fraktion aus Isophoron, höherkondensierten Produkten und Wasser und Katalysator, genannt Wertstoffstrom, wobei diese Fraktion anschließend einer Hydrolyse unterworfen wird.

22. Verfahren zur Herstellung von Isophoron nach mindestens einem der vorherigen Ansprüche, **dadurch gekennzeichnet, dass** die Fraktion a) als Brüdenstrom entnommen wird, enthaltend im Wesentlichen Aceton, Wasser und Leichtsiedern, im Wesentlichen Diacetonalkohol und Mesityloxid, kondensiert und dem Reaktor mit den Einsatzstoffen Aceton, Wasser und Katalysator wieder zugesetzt wird.

23. Verfahren zur Herstellung von Isophoron nach mindestens einem der vorherigen Ansprüche, **dadurch gekennzeichnet, dass** die Fraktion b) als Seitenstrom einer Destillationskolonne, bevorzugt einer Reaktivdestillationskolonne, entnommen wird.

24. Verfahren zur Herstellung von Isophoron nach mindestens einem der vorherigen Ansprüche, **dadurch gekennzeichnet, dass** die Aufreinigung der Fraktion b) durch eine Kombination aus Neutralisation oder Extraktion und anschließender Destillation in einer Reaktivdestillationskolonne, erfolgt,

25. Verfahren zur Herstellung von Isophoron nach mindestens einem der vorherigen Ansprüche, **dadurch gekennzeichnet, dass** die aufgearbeitete Phase der Fraktion b) mit den Wertprodukten aus Isophoron, Hochsiedern und gegebenenfalls Katalysator in die Hydrolyse geführt wird.

26. Verfahren zur Herstellung von Isophoron nach mindestens einem der vorherigen Ansprüche, **dadurch gekennzeichnet, dass** eine weitere erhaltene Phase der Fraktion b) aus Wertprodukten, im Wesentlichen enthaltend Aceton, Diacetonalkohol und Mesityloxid, in die Reaktion zurückgeführt wird.

27. Verfahren zur Herstellung von Isophoron nach mindestens einem der vorherigen Ansprüche, **dadurch gekennzeichnet, dass** in Stufe 3. der Wertstoffstrom, bevorzugt die Fraktion c), einer Hydrolyse unterworfen wird, bei der Nebenprodukte teilweise oder vollständig in Isophoron, Aceton und andere Wertprodukte überführt.

28. Verfahren zur Herstellung von Isophoron nach mindestens einem der vorherigen Ansprüche, **dadurch gekennzeichnet, dass** die Hydrolyse in einer Reaktivdestillationskolonne, durchgeführt wird, in dem die entstehenden Leichtsieder, im Wesentlichen enthaltend Aceton, Diacetonalkohol und Mesityloxid, direkt aus der Hydrolysezone entfernt und in die Reaktion zurückgeführt werden.

29. Verfahren zur Herstellung von Isophoron nach mindestens einem der vorherigen Ansprüche, **dadurch gekennzeichnet, dass** die Hydrolyse der Fraktion c) in einem Apparat, durch eine Reaktivdestillation, durchgeführt wird, bevorzugt in einer Reaktivdestillationskolonne, wobei gleichzeitig die Trennung des Reaktionsgemisches in die Fraktionen a) bis c) erfolgt, so dass die entstehenden Produkte gleichzeitig entsprechend aufgetrennt werden und die Fraktion c) hydrolysiert wird.

30. Verfahren zur Herstellung von Isophoron nach mindestens einem der vorherigen Ansprüche, **dadurch gekennzeichnet, dass** in Stufe 3. die Hydrolyse des Wertstoffstroms, bevorzugt die Fraktion c), bei Wasserkonzentration in der Hydrolyse beträgt dabei 0,1 - 99,9 Gew.-%, bevorzugt 30 - 90 Gew.-%, erfolgt.

31. Verfahren zur Herstellung von Isophoron nach mindestens einem der vorherigen Ansprüche, **dadurch gekennzeichnet, dass** in Stufe 3. die Hydrolyse des Wertstoffstroms, bevorzugt die Fraktion c), bei Katalysatorkonzentrationen von 0,001 - 10 Gew.-%, besonders bevorzugt von 0,05 - 1 Gew.-%, erfolgt.

32. Verfahren zur Herstellung von Isophoron nach mindestens einem der vorherigen Ansprüche, **dadurch gekennzeichnet, dass** in Stufe 3. die Hydrolyse des Wertstoffstroms, bevorzugt die Fraktion c), bei einem Druck im Hydrolysereaktor von 1 - 200 bar, bevorzugt 20 - 60 bar, besonders bevorzugt mindestens bei dem Druck, der auch im Isophoronsyntheseschritt (Reaktion) herrscht, durchgeführt wird.

33. Verfahren zur Herstellung von Isophoron nach mindestens einem der vorherigen Ansprüche, **dadurch gekennzeichnet, dass** in Stufe 3. die Hydrolyse des Wertstoffstroms, bevorzugt die Fraktion c), bei einer Temperatur von 100 - 300 °C, bevorzugt 210 - 260 °C durchgeführt wird.

34. Verfahren zur Herstellung von Isophoron nach mindestens einem der vorherigen Ansprüche, **dadurch gekennzeichnet, dass** in Stufe 3. die Hydrolyse des Wertstoffstroms, bevorzugt die Fraktion c), in einer Reaktivdestillationskolonne durchgeführt wird, wobei sich eine Temperatur oder ein Temperaturverlauf einstellt, der den Siedetemperaturen im Sumpf und auf den einzelnen Trenn- oder Reaktionsstufen entspricht.

35. Verfahren zur Herstellung von Isophoron nach mindestens einem der vorherigen Ansprüche, **dadurch gekennzeichnet, dass** in Stufe 3. die aufgearbeitete Fraktion des Wertstoffstroms, bevorzugt der Fraktion c), einer Phasentrennung unterworfen wird, in eine im Wesentlichen organische Fraktion, bevorzugt Fraktion d), und eine im Wesentlichen wässrige Fraktion, bevorzugt Fraktion e).

36. Verfahren zur Herstellung von Isophoron nach mindestens einem der vorherigen Ansprüche, **dadurch gekennzeichnet, dass** in Stufe 3. die Aufreinigung der organische Fraktion, bevorzugt Fraktion d), durch Destillation, besonders bevorzugt eine Kombination aus Neutralisation oder Extraktion und folgender Destillation, erfolgt.

37. Verfahren zur Herstellung von Isophoron nach mindestens einem der vorherigen Ansprüche, **dadurch gekennzeichnet, dass** in Stufe 4. die wässrige Fraktion, bevorzugt Fraktion e), einer Abwasseraufreinigung zugeführt wird, wobei die Abwasseraufreinigung bevorzugt in einer oder mehreren Destillationskolonnen durchgeführt wird.

38. Verfahren zur Herstellung von Isophoron nach mindestens einem der vorherigen Ansprüche, **dadurch gekennzeichnet, dass** der Druck bei der Abwasseraufreinigung in Stufe 4. in der Abwasserkolonne 1 - 200 bar, bevorzugt 20 - 60 bar, beträgt.

39. Verfahren zur Herstellung von Isophoron nach mindestens einem der vorherigen Ansprüche, **dadurch gekennzeichnet, dass** in Stufe 4. bei dem Systemdruck in der Abwasserkolonne gearbeitet wird, der sich im Gesamtsystem Hydrolyse/Abwasserkolonne einstellt, bevorzugt wenn die Brüden der Abwasserkolonne direkt in den Hydrolyseteil der Reaktivdestillationskolonne geleitet werden.

40. Verfahren zur Herstellung von Isophoron nach mindestens einem der vorherigen Ansprüche, **dadurch gekennzeichnet, dass** in Stufe 5. der Druck in der Enspannungsverdampfung unterhalb des Druckes in der Abwasserkolonne liegt.

41. Verfahren zur Herstellung von Isophoron nach mindestens einem der vorherigen Ansprüche, **dadurch gekennzeichnet, dass** in Stufe 5. die Brüden g) der Entspannungverdampfungsstufe, die im Wesentlichen aus reinem Wasser bestehen, kondensiert und als Wasser in die Reaktion zurückgeführt werden.

## Claims

1. Process for preparing isophorone by
1. catalysed aldol condensations with acetone as a reactant,
2. workup of the reaction product,
3. hydrolysis of the stream of value and separation into an organic fraction and an aqueous fraction,
obtaining isophorone from the organic fraction,
4. distillative workup of the aqueous fraction in the presence of at least one antifoam and passing the vapours from the top of the distillative workup apparatus onward into the hydrolysis apparatus,
**characterized in that** polyether polyols are used as antifoam.

2. Process for preparing isophorone according to Claim 1, **characterized in that**
5. the water from the bottoms of the distillative workup of the aqueous fraction is subjected to a flash evaporation and the purified water which forms is recycled into the process for preparing isophorone.

3. Process for preparing isophorone according to at least one of the preceding claims, **characterized in that** the antifoams used are mineral oils, natural oils, higher alcohols, polyols, polyether polyols, silicones, or any desired mixtures of the components mentioned.

4. Process for preparing isophorone, **characterized in that** an aqueous mixture of at least one antifoam is used.

5. Process for preparing isophorone according to at least one of the preceding claims, **characterized in that** an aqueous mixture of at least one polyether polyol is used.

6. Process for preparing isophorone according to at least one of the preceding claims, **characterized in that** 0.00005 to 0.5% by weight of antifoam, based on the chemical substance, i.e. on the antifoam, is present in the wastewater column of stage 4., preferably < 0.1% by weight, more preferably < 0.01% by weight.

7. Process for preparing isophorone according to at least one of the preceding claims, **characterized in that** at least one antifoam is added to the feed of a wastewater column of the distillative workup of the aqueous fraction.

8. Process for preparing isophorone according to at least one of the preceding claims, **characterized in that** the antifoam is introduced into the feed of the wastewater column in such a way that the total amount of antifoam is < 0.5% by weight, preferably < 0.1% by weight and more preferably < 0.01% by weight in the wastewater column, based on the chemical substance, i.e. on the antifoam.

9. Process for preparing isophorone according to at least one of the preceding Claims 1-8, **characterized in that** the reaction in stage 1. is performed in the liquid phase, the acetone being converted to isophorone by catalytic reaction at temperatures in the range from 100 to 250°C, preferably 150 - 250°C and more preferably 180 - 250°C, and a pressure range of 5 to 50 bar, preferably 10 - 50 bar and more preferably of 20 - 50 bar, it being possible to combine the values specified as desired.

10. Process for preparing isophorone according to at least one of the preceding Claims 1-8, **characterized in that** the reaction in stage 1. is performed in the gas phase, the acetone being converted to isophorone by catalytic reaction at temperatures in the range from 100 to 400°C and preferably 200-400°C.

11. Process for preparing isophorone according to at least one of the preceding Claims 1-8, **characterized in that** the reaction in stage 1. is performed in the supercritical range, the acetone being converted to isophorone by catalytic reaction at temperatures in the range from 250 to 350°C and a pressure range of 50 to 200 bar.

12. Process for preparing isophorone according to at least one of the preceding claims, **characterized in that** a homogeneous or heterogeneous catalyst, preferably a homogeneous catalyst, is used in stage 1.

13. Process for preparing isophorone according to Claim 9, **characterized in that** the reaction is effected in the liquid phase with a homogeneous catalyst.

14. Process for preparing isophorone according to Claim 13, **characterized in that** amounts of alkali of < 1% by weight, preferably of < 0.2% by weight, are used as a catalyst, preferably NaOH in amounts of 0.015 to 0.05% by weight.

15. Process for preparing isophorone according to at least one of the preceding claims, **characterized in that** the reaction in stage 1. is performed in reactors selected from tubular reactors, stirred tanks, stirred tank cascades, fixed bed reactors, reactive distillation columns, microstructured reactors, loop reactors, or in combinations of these reactors.

16. Process for preparing isophorone according to at least one of the preceding claims, **characterized in that** the reaction in stage 1. is performed in reactive distillation columns, tubular reactors or fixed bed reactors, more preferably in a tubular reactor.

17. Process for preparing isophorone according to at least one of the preceding claims, **characterized in that** performance of the reaction is followed by workup of the reaction mixture in stage 2. and separation into the individual components, the removal being performed in full or in part.

18. Process for preparing isophorone according to at least one of the preceding claims, **characterized in that** the removal of the individual fractions in stage 2. is performed by separation methods such as distillation, flash evaporation, crystallization, extraction, sorption, permeation, phase separation or combinations of the above, continuously or batchwise, in one or more stages, preferably by distillation in one or more apparatuses.

19. Process for preparing isophorone according to at least one of the preceding claims, **characterized in that** the distillation in stage 2. is performed spatially separately from the isophorone synthesis or takes place in a reaction apparatus, preferably by means of a reactive distillation, preferably in a reactive distillation column.

20. Process for preparing isophorone according to at least one of the preceding claims, **characterized in that** the removal in stage 2. is performed spatially separately from the isophorone synthesis by a reactive distillation, preferably in a reactive distillation column, with a sidestream withdrawal.

21. Process for preparing isophorone according to at least one of the preceding claims, **characterized in that** the removal in stage 2. is effected in three fractions:
a) a fraction composed of unconverted acetone, water and low boilers, which is condensed and then recycled into the reactor for reaction;
b) a fraction in which coloured substances in particular are enriched, this fraction being purified further and the materials of value present being recycled into the process;
c) a fraction composed of isophorone, more highly condensed products and water and catalyst, called material of value stream, this fraction subsequently being subjected to a hydrolysis.

22. Process for preparing isophorone according to at least one of the preceding claims, **characterized in that** fraction a) is withdrawn as a vapour stream comprising essentially acetone, water and low boilers, essentially diacetone alcohol and mesityl oxide, condensed and added again to the reactor with the acetone, water and catalyst feedstocks.

23. Process for preparing isophorone according to at least one of the preceding claims, **characterized in that** fraction b) is withdrawn as a sidestream of a distillation column, preferably of a reactive distillation column.

24. Process for preparing isophorone according to at least one of the preceding claims, **characterized in that** fraction b) is purified by a combination of neutralization or extraction and subsequent distillation in a reactive distillation column.

25. Process for preparing isophorone according to at least one of the preceding claims, **characterized in that** the worked-up phase of fraction b) is conducted into the hydrolysis with the products of value composed of isophorone and high boilers, with or without catalyst.

26. Process for preparing isophorone according to at least one of the preceding claims, **characterized in that** any further phase of fraction b) obtained, composed of products of value essentially comprising acetone, diacetone alcohol and mesityl oxide, is recycled into the reaction.

27. Process for preparing isophorone according to at least one of the preceding claims, **characterized in that** the material of value stream, preferably fraction c), is subjected in stage 3. to a hydrolysis in which by-products are converted partly or fully to isophorone, acetone and other products of value.

28. Process for preparing isophorone according to at least one of the preceding claims, **characterized in that** the hydrolysis is performed in a reactive distillation column in which the low boilers formed, essentially comprising acetone, diacetone alcohol and mesityl oxide, are removed directly from the hydrolysis zone and recycled into the reaction.

29. Process for preparing isophorone according to at least one of the preceding claims, **characterized in that** the hydrolysis of fraction c) is performed in an apparatus, by a reactive distillation, preferably in a reactive distillation column, with simultaneous separation of the reaction mixture into fractions a) to c), such that the products formed are correspondingly separated at the same time as fraction c) is hydrolyzed.

30. Process for preparing isophorone according to at least one of the preceding claims, **characterized in that** the hydrolysis of the material of value stream, preferably fraction c), in stage 3. is effected at water concentrations of 0.1 - 99.9% by weight, preferably 30 - 90% by weight.

31. Process for preparing isophorone according to at least one of the preceding claims, **characterized in that** the hydrolysis of the material of value stream, preferably fraction c), in stage 3. is effected at catalyst concentrations of 0.001 - 10% by weight, more preferably of 0.05 - 1% by weight.

32. Process for preparing isophorone according to at least one of the preceding claims, **characterized in that** the hydrolysis of the material of value stream, preferably fraction c), is performed in stage 3. at a pressure in the hydrolysis reactor of 1 - 200 bar, preferably 20 - 60 bar, more preferably at least at the pressure which also exists in the isophorone synthesis step (reaction).

33. Process for preparing isophorone according to at least one of the preceding claims, **characterized in that** the hydrolysis of the material of value stream, preferably fraction c), in stage 3. is effected at a temperature of 100 - 300°C, preferably 210 - 260°C.

34. Process for preparing isophorone according to at least one of the preceding claims, **characterized in that** the hydrolysis of the material of value stream, preferably fraction c), is performed in stage 3. in a reactive distillation column, with establishment of a temperature or temperature profile according to the boiling temperatures in the bottoms and at the individual separation or reaction stages.

35. Process for preparing isophorone according to at least one of the preceding claims, **characterized in that** the worked-up fraction of the material of value stream, preferably fraction c), is subjected in stage 3. to a phase separation into an essentially organic fraction, preferably fraction d), and an essentially aqueous fraction, preferably fraction e).

36. Process for preparing isophorone according to at least one of the preceding claims, **characterized in that** the organic fraction, preferably fraction d), is purified in stage 3. by distillation, more preferably a combination of neutralization or extraction and subsequent distillation.

37. Process for preparing isophorone according to at least one of the preceding claims, **characterized in that** the aqueous fraction, preferably fraction e), is supplied in stage 4. to a wastewater cleaning operation, the wastewater cleaning operation preferably being performed in one or more distillation columns.

38. Process for preparing isophorone according to at least one of the preceding claims, **characterized in that** the pressure in the wastewater cleaning operation in stage 4. in the wastewater column is 1 - 200 bar, preferably 20 - 60 bar.

39. Process for preparing isophorone according to at least one of the preceding claims, **characterized in that** operation in stage 4. is effected at the system pressure in the wastewater column which is established in the overall hydrolysis/wastewater column system, preferably when the vapours of the wastewater column are passed directly into the hydrolysis section of the reactive distillation column.

40. Process for preparing isophorone according to at least one of the preceding claims, **characterized in that** the pressure in stage 5. in the flash evaporation is below the pressure in the wastewater column.

41. Process for preparing isophorone according to at least one of the preceding claims, **characterized in that** the vapours g) of the flash evaporation stage consisting essentially of pure water are condensed in stage 5. and recycled as water into the reaction.

## Revendications

1. Procédé de fabrication d'isophorone par
1. condensation d'aldol catalysée avec de l'acétone en tant que réactif,
2. traitement du produit de réaction,
3. hydrolyse du courant de valeur et séparation en une fraction organique et une fraction aqueuse,
obtention d'isophorone à partir de la fraction organique,
4. traitement par distillation de la fraction aqueuse en présence d'au moins un antimousse et transfert des vapeurs depuis la tête de l'appareil du traitement par distillation vers l'appareil d'hydrolyse,
**caractérisé en ce que** des polyéther-polyols sont utilisés en tant qu'antimousse.

2. Procédé de fabrication d'isophorone selon la revendication 1, **caractérisé en ce que**
5. l'eau issue du fond du traitement par distillation de la fraction aqueuse est soumise à une évaporation par détente et l'eau purifiée formée est recyclée dans le procédé de fabrication d'isophorone.

3. Procédé de fabrication d'isophorone selon au moins l'une quelconque des revendications précédentes, **caractérisé en ce que** des huiles minérales, des huiles naturelles, des alcools supérieurs, des polyols, des polyéther-polyols, des silicones ou des mélanges quelconques des composants cités sont utilisés en tant qu'antimousse.

4. Procédé de fabrication d'isophorone, **caractérisé en ce qu'**un mélange aqueux d'au moins un antimousse est utilisé.

5. Procédé de fabrication d'isophorone selon au moins l'une quelconque des revendications précédentes, **caractérisé en ce qu'**un mélange aqueux d'au moins un polyéther-polyol est utilisé.

6. Procédé de fabrication d'isophorone selon au moins l'une quelconque des revendications précédentes, **caractérisé en ce que** 0,00005 à 0,5 % en poids d'un antimousse, par rapport à la substance chimique, c'est-à-dire par rapport à l'antimousse, est contenu dans la colonne à eau résiduaire de l'étape 4, de préférence < 0,1 % en poids, de manière particulièrement préférée < 0,01 % en poids.

7. Procédé de fabrication d'isophorone selon au moins l'une quelconque des revendications précédentes, **caractérisé en ce que** le traitement par distillation de la fraction aqueuse a lieu par l'ajout d'au moins un antimousse dans l'alimentation d'une colonne à eau résiduaire.

8. Procédé de fabrication d'isophorone selon au moins l'une quelconque des revendications précédentes, **caractérisé en ce que** l'antimousse est introduit dans l'alimentation de la colonne à eau résiduaire de sorte que la quantité totale d'antimousse soit < 0,5 % en poids, de préférence < 0,1 % en poids, de manière particulièrement préférée < 0,01 % en poids, dans la colonne à eau résiduaire, par rapport à la substance chimique, c'est-à-dire par rapport à l'antimousse.

9. Procédé de fabrication d'isophorone selon au moins l'une quelconque des revendications 1 à 8 précédentes, **caractérisé en ce que** la réalisation de la réaction à l'étape 1. a lieu en phase liquide, l'acétone étant transformée en isophorone par une réaction catalytique à des températures dans la plage allant de 100 à 250 °C, de préférence de 150 à 250 °C, de manière particulièrement préférée de 180 à 250 °C, et dans une plage de pressions allant de 5 à 50 bar, de préférence de 10 à 50 bar, de manière particulièrement préférée de 20 à 50 bar, les valeurs indiquées pouvant être combinées de manière quelconque les unes avec les autres.

10. Procédé de fabrication d'isophorone selon au moins l'une quelconque des revendications 1 à 8 précédentes, **caractérisé en ce que** la réalisation de la réaction à l'étape 1. a lieu en phase gazeuse, l'acétone étant transformée en isophorone par une réaction catalytique à des températures dans la plage allant de 100 à 400 °C, de préférence de 200 à 400 °C.

11. Procédé de fabrication d'isophorone selon au moins l'une quelconque des revendications 1 à 8 précédentes, **caractérisé en ce que** la réalisation de la réaction à l'étape 1. a lieu dans la plage supercritique, l'acétone étant transformée en isophorone par une réaction catalytique à des températures dans la plage allant de 250 à 350 °C et dans une plage de pressions allant de 50 à 200 bar.

12. Procédé de fabrication d'isophorone selon au moins l'une quelconque des revendications précédentes, **caractérisé en ce qu'**un catalyseur homogène ou hétérogène, de préférence un catalyseur homogène, est utilisé à l'étape 1.

13. Procédé de fabrication d'isophorone selon la revendication 9, **caractérisé en ce que** la réaction a lieu en phase liquide avec un catalyseur homogène.

14. Procédé de fabrication d'isophorone selon la revendication 13, **caractérisé en ce que** des quantités d'alcalis < 1 % en poids, de préférence < 0,2 % en poids, sont utilisées en tant que catalyseur, de préférence NaOH en quantités de 0,015 à 0,05 % en poids.

15. Procédé de fabrication d'isophorone selon au moins l'une quelconque des revendications précédentes, **caractérisé en ce que** la réaction à l'étape 1. est réalisée dans des réacteurs choisis parmi des réacteurs tubulaires, des cuves agitées, des cascades de cuves agitées, des réacteurs à lit fixe, des colonnes de distillation réactives, des réacteurs microstructurés, des réacteurs à boucle ou dans des combinaisons de ces réacteurs.

16. Procédé de fabrication d'isophorone selon au moins l'une quelconque des revendications précédentes, **caractérisé en ce que** la réaction à l'étape 1. est réalisée dans des colonnes de distillation réactives, des réacteurs tubulaires ou des réacteurs à lit fixe, de manière particulièrement préférée dans un réacteur tubulaire.

17. Procédé de fabrication d'isophorone selon au moins l'une quelconque des revendications précédentes, **caractérisé en ce qu'**après la réalisation de la réaction, le mélange réactionnel est traité à l'étape 2. et les composants individuels sont séparés, la séparation étant réalisée en totalité ou en partie.

18. Procédé de fabrication d'isophorone selon au moins l'une quelconque des revendications précédentes, **caractérisé en ce qu'**à l'étape 2., la séparation des fractions individuelles est réalisée par des méthodes de séparation telles que la distillation, l'évaporation par détente, la cristallisation, l'extraction, la sorption, la perméation, la séparation de phases ou des combinaisons des méthodes citées, de manière continue ou discontinue, en une ou plusieurs étapes, de préférence par distillation dans un ou plusieurs appareils.

19. Procédé de fabrication d'isophorone selon au moins l'une quelconque des revendications précédentes, **caractérisé en ce qu'**à l'étape 2., la distillation est réalisée de manière séparée dans l'espace de la synthèse d'isophorone ou a lieu dans un appareil de réaction, de préférence par une distillation réactive, de préférence dans une colonne de distillation réactive.

20. Procédé de fabrication d'isophorone selon au moins l'une quelconque des revendications précédentes, **caractérisé en ce qu'**à l'étape 2., la séparation est réalisée de manière séparée dans l'espace de la synthèse d'isophorone par une distillation réactive, de préférence dans une colonne de distillation réactive, avec un soutirage de courant latéral.

21. Procédé de fabrication d'isophorone selon au moins l'une quelconque des revendications précédentes, **caractérisé en ce qu'**à l'étape 2., la séparation a lieu en trois fractions :
a) une fraction constituée d'acétone non réagie, d'eau et de composants de point d'ébullition faible, qui est condensée, puis recyclée dans la réaction dans le réacteur ;
b) une fraction, qui est notamment enrichie en substances colorantes, cette fraction étant davantage purifiée et les substances de valeur contenues étant recyclées dans le procédé ;
c) une fraction constituée d'isophorone, de produits condensés supérieurs et d'eau et de catalyseur, nommée courant de valeur, cette fraction étant ensuite soumise à une hydrolyse.

22. Procédé de fabrication d'isophorone selon au moins l'une quelconque des revendications précédentes, **caractérisé en ce que** la fraction a) est soutirée en tant que courant de vapeur, contenant principalement de l'acétone, de l'eau et des composés de point d'ébullition faible, principalement de l'alcool diacétonique et de l'oxyde de mésityle, condensée et réintroduite dans le réacteur avec les matières premières acétone, eau et catalyseur.

23. Procédé de fabrication d'isophorone selon au moins l'une quelconque des revendications précédentes, **caractérisé en ce que** la fraction b) est soutirée en tant que courant latéral d'une colonne de distillation, de préférence d'une colonne de distillation réactive.

24. Procédé de fabrication d'isophorone selon au moins l'une quelconque des revendications précédentes, **caractérisé en ce que** la purification de la fraction b) a lieu par une combinaison d'une neutralisation ou d'une extraction et d'une distillation ultérieure dans une colonne de distillation réactive.

25. Procédé de fabrication d'isophorone selon au moins l'une quelconque des revendications précédentes, **caractérisé en ce que** la phase traitée de la fraction b) est introduite avec les produits de valeur constitués par l'isophorone, les composés de point d'ébullition élevé et éventuellement le catalyseur, dans l'hydrolyse.

26. Procédé de fabrication d'isophorone selon au moins l'une quelconque des revendications précédentes, **caractérisé en ce qu'**une autre phase obtenue de la fraction b), constituée de produits de valeur, contenant principalement de l'acétone, de l'alcool diacétonique et de l'oxyde de mésityle, est recyclée dans la réaction.

27. Procédé de fabrication d'isophorone selon au moins l'une quelconque des revendications précédentes, **caractérisé en ce qu'**à l'étape 3., le courant de valeur, de préférence la fraction c), est soumis à une hydrolyse, lors de laquelle les produits secondaires sont transformés en partie ou en totalité en isophorone,, acétone et autres produits de valeur.

28. Procédé de fabrication d'isophorone selon au moins l'une quelconque des revendications précédentes, **caractérisé en ce que** l'hydrolyse est réalisée dans une colonne de distillation réactive, dans laquelle les composés de point d'ébullition faible formés, contenant principalement de l'acétone, de l'alcool diacétonique et de l'oxyde de mésityle, sont éliminés directement de la zone d'hydrolyse et recyclés dans la réaction.

29. Procédé de fabrication d'isophorone selon au moins l'une quelconque des revendications précédentes, **caractérisé en ce que** l'hydrolyse de la fraction c) est réalisée dans un appareil, par une distillation réactive, de préférence dans une colonne de distillation réactive, la séparation du mélange réactionnel en les fractions a) à c) ayant lieu simultanément, de sorte que les produits formés soient séparés simultanément de manière appropriée et que la fraction c) soit hydrolysée.

30. Procédé de fabrication d'isophorone selon au moins l'une quelconque des revendications précédentes, **caractérisé en ce qu'**à l'étape 3., l'hydrolyse du courant de valeur, de préférence la fraction c), a lieu à des concentrations en eau de 0,1 à 99,9 % en poids, de préférence de 30 à 90 % en poids.

31. Procédé de fabrication d'isophorone selon au moins l'une quelconque des revendications précédentes, **caractérisé en ce qu'**à l'étape 3., l'hydrolyse du courant de valeur, de préférence la fraction c), a lieu à des concentrations de catalyseur de 0,001 à 10 % en poids, de manière particulièrement préférée de 0,05 à 1 % en poids.

32. Procédé de fabrication d'isophorone selon au moins l'une quelconque des revendications précédentes, **caractérisé en ce qu'**à l'étape 3., l'hydrolyse du courant de valeur, de préférence la fraction c), est réalisée à une pression dans le réacteur d'hydrolyse de 1 à 200 bar, de préférence de 20 à 60 bar, de manière particulièrement préférée à la pression qui règne également dans l'étape de synthèse d'isophorone (réaction).

33. Procédé de fabrication d'isophorone selon au moins l'une quelconque des revendications précédentes, **caractérisé en ce qu'**à l'étape 3., l'hydrolyse du courant de valeur, de préférence la fraction c), est réalisée à une température de 100 à 300 °C, de préférence de 210 à 260 °C.

34. Procédé de fabrication d'isophorone selon au moins l'une quelconque des revendications précédentes, **caractérisé en ce qu'**à l'étape 3., l'hydrolyse du courant de valeur, de préférence la fraction c), est réalisée dans une colonne de distillation réactive, une température ou un gradient de température qui correspond aux températures d'ébullition dans le fond et dans les étapes de séparation ou de réaction individuelles s'ajustant.

35. Procédé de fabrication d'isophorone selon au moins l'une quelconque des revendications précédentes, **caractérisé en ce qu'**à l'étape 3., la fraction traitée du courant de valeur, de préférence la fraction c), est soumise à une séparation de phases, en une fraction essentiellement organique, de préférence la fraction d), et une fraction essentiellement aqueuse, de préférence la fraction e).

36. Procédé de fabrication d'isophorone selon au moins l'une quelconque des revendications précédentes, **caractérisé en ce qu'**à l'étape 3., la purification de la fraction organique, de préférence la fraction d), a lieu par distillation, de manière particulièrement préférée une combinaison d'une neutralisation ou d'une extraction et d'une distillation ultérieure.

37. Procédé de fabrication d'isophorone selon au moins l'une quelconque des revendications précédentes, **caractérisé en ce qu'**à l'étape 4., la fraction aqueuse, de préférence la fraction e), est introduite dans une purification de l'eau résiduaire, la purification de l'eau résiduaire étant de préférence réalisée dans une ou plusieurs colonnes de distillation.

38. Procédé de fabrication d'isophorone selon au moins l'une quelconque des revendications précédentes, **caractérisé en ce que** la pression lors de la purification de l'eau résiduaire à l'étape 4. dans la colonne à eau résiduaire est de 1 à 200 bar, de préférence de 20 à 60 bar.

39. Procédé de fabrication d'isophorone selon au moins l'une quelconque des revendications précédentes, **caractérisé en ce que** l'étape 4. est réalisée à la pression du système dans la colonne à eau résiduaire, qui s'ajuste dans l'ensemble du système hydrolyse/colonne à eau résiduaire, de préférence lorsque les vapeurs de la colonne à eau résiduaire sont introduites directement dans la partie d'hydrolyse de la colonne de distillation réactive.

40. Procédé de fabrication d'isophorone selon au moins l'une quelconque des revendications précédentes, **caractérisé en ce qu'**à l'étape 5., la pression dans l'évaporation par détente est inférieure à la pression dans la colonne à eau résiduaire.

41. Procédé de fabrication d'isophorone selon au moins l'une quelconque des revendications précédentes, **caractérisé en ce qu'**à l'étape 5., les vapeurs g) de l'étape d'évaporation par détente, qui sont essentiellement constituées d'eau pure, sont condensées et recyclées dans la réaction en tant qu'eau.
